(19) Europäisches Patentamt
European Patent Office
Office européen des brevets

(11) **EP 3 138 932 B1**

(12) **EUROPÄISCHE PATENTSCHRIFT**

(45) Veröffentlichungstag und Bekanntmachung des Hinweises auf die Patenterteilung:
**14.11.2018 Patentblatt 2018/46**

(51) Int Cl.:
*C22B 3/02* *(2006.01)*    *C01G 41/02* *(2006.01)*
*C22B 3/18* *(2006.01)*    *C22B 7/00* *(2006.01)*
*C22B 34/36* *(2006.01)*

(21) Anmeldenummer: **15002564.1**

(22) Anmeldetag: **01.09.2015**

(54) **VERFAHREN UND VORRICHTUNG ZUR GEWINNUNG EINES PULVERS AUS PARTIKELN VON WOLFRAM ODER WOLFRAMVERBINDUNGEN MIT EINER PARTIKELGRÖSSE IM NANO-, MIKRON- ODER SUBMIKRONBEREICH**

METHOD AND DEVICE FOR OBTAINING A POWDER FROM PARTICLES OF TUNGSTEN OR TUNGSTEN COMPOUNDS WITH A SIZE IN THE NANO-, MICRON OR SUBMICRON RANGE

PROCEDE ET DISPOSITIF DE PRODUCTION D'UNE POUDRE A PARTIR DE PARTICULES DE TUNGSTENE OU DE LIAISONS DE TUNGSTENE AYANT UNE GRANDEUR DE PARTICULE SITUEE DANS LA PLAGE DE NANO, MICRO OU SUBMICRON

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**08.03.2017 Patentblatt 2017/10**

(73) Patentinhaber: **Galuga, Andrey**
**90449 Nürnberg (DE)**

(72) Erfinder:
• **Galuga, Andrey**
**90449 Nürnberg (DE)**
• **Baravov, Georgij**
**Sevastopol 299098 (RU)**
• **Gavrish, Vladimir**
**Sevastopol 299029 (RU)**
• **Smirnov, Sergei**
**Sevastopol 299055 (RU)**
• **Losenkov, Aleksandr**
**St. Petersburg 196135 (RU)**
• **Vostrognutov, Stanislav**
**Severodvinsk 164524 (RU)**

(74) Vertreter: **Küchler, Stefan**
**Patentanwalt**
**Färberstrasse 20**
**90402 Nürnberg (DE)**

(56) Entgegenhaltungen:
DE-A1-102011 000 955    JP-A- 2009 249 415
JP-A- 2013 204 068    US-A- 5 831 123
US-A1- 2007 048 516

• KOJIMA T ET AL: "Recycling process of WC-Co cermets by hydrothermal treatment", JOURNAL OF MATERIALS SCIENCE, KLUWER ACADEMIC PUBLISHERS, BO, Bd. 40, Nr. 19, 1. Oktober 2005 (2005-10-01), Seiten 5167-5172, XP019210776, ISSN: 1573-4803, DOI: 10.1007/S10853-005-4407-0
• DEBARAJ MISHRA AND YOUNG HA RHEE: "Microbial Leaching of Metals from Solid Industrial Waste", JOURNAL OF MICROBIOLOGY, Bd. 52, Nr. 1, 2014, Seiten 1-7, XP002754353,

**Beschreibung**

Die Erfindung betrifft eine Technologie zur Herstellung von Metall im Nanobereich und im Submikronbereich, insbesondere für ein Pulver aus Wolfram, Wolframcarbid, Carbiden, Feststofflösungen von Wolfram, Titan und/oder Tantal, beim Recycling von Produkten und festen Wolframschwerlegierungen. Die Erfindung kann zur Herstellung von Metalllegierungen aus pulvermetallurgischen Materialien verwendet werden, als nanogroße Füllstoffe für radioaktive Metallopolymere, für Materialien mit einer hohen photokatalytischen Leistung, aufgrund der hohen Härte der Maschinenteile beim Schweißen, Sprühen.

Insbesondere richtet sich die Erfindung auf ein Verfahren und eine Vorrichtung zur Gewinnung eines Pulvers aus Partikeln von Wolfram oder Wolframverbindungen mit einer Partikelgröße im Nano-, Mikron- oder Submikronbereich, insbesondere von W, $WO_3$, WC oder WC-TiC, WC-TaC oder WC-TiC-TaC, unter Recyceln von Produkten aus Schwermetall- oder Hartmetall-Legierungen in wenigstens einem eine Dispersionsflüssigkeit enthaltenden Reaktor-Behälter.

Moderne Materialien mit Partikelgrößen im Nano- und Submikronbereich haben einen weiten Anwendungsbereich. Es wurde festgestellt, dass die Leistungsfähigkeit von Carbid durch Verringerung der Kristallgröße von 300 nm bis 500 nm deutlich verbessert wurde. Nanopulver finden Anwendung als Füllstoffe, um neue Metallpolymere mit einzigartigen Eigenschaften herzustellen, wie auch bei der Herstellung von radioaktive Strahlung abschirmenden Materialien und von Materialien mit hohen photokatalytischen Eigenschaften im sichtbaren Wellenlängenbereich von 380 nm bis 800 nm.

Etwa 30 % des Weltverbrauchs an Wolfram und seinen Legierungen wird durch Recycling bereitgestellt. Derzeit gibt es mehrere globale, industrielle Behandlungen für Wolfram-Sinterhartlegierungs-Abfall, um daraus ein Pulver für die weitere Nutzung (in der Regel für recycelte Sinterhartmetalle) zu erhalten.

Im Rahmen von bekannten Arten der Nutzung und des Mahlens von gesintertem Hartlegierungspulver wird der feste Abfall erhitzt, jedoch nicht unterhalb der Solidustemperatur der Legierung und nicht höher als 1800 °C, und direkt nach dem Erhitzen erfolgt das Schleifen. Ferner wird das Erhitzen des Materials in einem Ofen mit einer Schutzatmosphäre durchgeführt. Nachteile dieses Verfahrens sind die hohen Energiekosten, die Notwendigkeit einer Kugelmühle für das Mahlen zum Nanopulver; auch gelingt das Recycling nur aus Wolfram-Kobalt-Legierungen und aus Legierungen und festen Lösungen von Wolfram, Titan und/oder Tantal. Ein weiterer Nachteil ist die Tatsache, dass das erhaltenen Pulver die gleiche chemische Zusammensetzung wie das Ausgangsmaterial hat.

Bei einem bekannten Verfahren erfolgt das Recyceln des Pulvers aus Wolframcarbid in Form von Schlamm. Das Verfahren umfasst das Eintauchen der Abfälle in einen sauren Elektrolyt, Durchführen einer Elektrolyse in einer Lösung mit dem Transfer von Kobalt und Wolfram aus der Aufschlämmung. Diese Elektrolyse wird mit Gleichstrom durchgeführt. Der Elektrolyt ist 5- bis 15-prozentige Schwefelsäurelösung, sowie 2 % eines Reduktionsmittels. Die resultierende Aufschlämmung wird in der Form eines Metallpulvers aus Wolfram erhalten. Nachteile dieser Methode sind der hohe Energieverbrauch, ferner die fehlende Möglichkeit der Gewinnung von Nanopulvern, sowie die Einschränkung des Verfahrens ausschließlich auf eine Wolfram-KobaltLegierung.

Ferner gibt es ein Verfahren zur Gewinnung von Nanopulver mit einem Hartmetallrecyclingprozess. Wolframcarbid-Schrott wird einer elektroerosiven Dispersion in destilliertem Wasser bei einer Spannung zwischen den Elektroden von 100 V bis 160 V und einer Impulsfolgefrequenz von 140 Hz bis 660 Hz unterzogen, um ein Pulver zu erhalten, aus dessen Teilchen sich Nanopartikel abtrennen lassen. Der Nachteil dieses Verfahrens ist die Tatsache, dass die im Rahmen der Pulvermaterialien erhaltenen Teilchen eine im Wesentlichen kugelförmige Partikelform haben, ferner eine Vielfalt von Größen in einem Bereich von 0,001 Mikron bis 100 Mikron, bei der Anwendung dieses Verfahrens auf Wolframkarbid-Kobalt.

Die Offenlegungsschrift DE 10 2011 000 955 A1 offenbart ein Verfahren zur Rückgewinnung von Hartstoffpartikeln, die in einer in schütt- oder rieselfähiger Form vorliegenden Reststoffmenge eines Hartmetalls vorhanden sind, das eine aus einem Stahl, aus Nickel oder aus einer Nickellegierung bestehende Matrix aufweist, in der die Hartstoffpartikel eingelagert sind; dieses Verfahren umfasst die Arbeitsschritte

- Einfüllen der Reststoffmenge in ein Säurebad, das eine starke Säure mit einem bei Raumtemperatur gemessenen $pK_5$-Wert < 4 enthält,
- Zugabe eines Oxidationsmittels zu dem Säurebad,
- Auflösen der Matrix der Reststoffmenge,
- Abscheiden der nach dem Auflösen der Matrix in dem Säurebad enthaltenen Hartstoffpartikel.

Hier wird nur die Matrix der Reststoffmenge aufgelöst; die darin enthaltenen Hartstoffpartikel werden dagegen nicht verändert. Die Partikelgröße richtet sich daher nach der Partikelgröße in dem eingesetzten Material und kann nicht beeinflusst werden.

Die Veröffentlichung Kojima T. et al.; "Recycling process of WC-Co cermets by hydrothermal treatment", in Journal of Materials Science, Bd. 40, Nr. 19, Seiten 5167 - 5172, beschreibt nur Parameter eines Recycling-Verfahrens für WC-Co-Cermets durch eine hydrothermale Behandlung, aber keinen dafür zu verwendenden Behälter. Die Partikelgröße kann dabei durch einen abschließenden Mahlvorgang beeinflusst werden. Jedoch ist das Mahlen auf Nanopartikelgröße sehr aufwändig.

Die JP 2009 249 415 A beschreibt ein Verfahren und eine dabei verwendbare Vorrichtung zur Herstellung eines pulverigen Polymers unter Zerstoßen oder Mahlen eines Granulats in einer Trommel; dazu wird eine rotierende Trommeleinheit verwendet, die auf einem chassisartigen Ständer-Rahmen-Gerüst drehbar abgestützt ist. Dieses chassisartige Ständer-Rahmen-Gerüst kann gegenüber der Horizontalen geneigt sein, unter Verwendung eines manuell betätigbaren Hebers, welcher auf einem horizontalen Untergrund angeordnet ist. Dieser Heber kann entweder unveränderbar sein oder verstellbar, er dient aber stets nur zur Einstellung des Neigungswinkels, welcher sodann während des chemischen Prozesses konstant bleibt. Aufgrund seiner einmalig eingestellten Neigung gegenüber der Horizontalen wird sich das zu zerstoßende bzw. zu ermahlende Granulat bald an einem Ende der Trommel ansammeln und dort die Trommel alsbald verlassen. Der eigentliche Mahlvorgang ist mit einer derartigen Anordnung demnach sehr kurz und kann daher keine Nanopartikel erzeugen.

Das Problem der Erfindung ist die Entwicklung von Design-Lösungen und Formen der verwendeten Behälter, um eine hohe Ausbeute an nanoskaligem und/oder submikrongroßen Pulver aus Wolfram oder Wolframkarbid zu erhalten, sowie ein Nanopulver aus festen Lösungen von Carbiden mit Wolfram, Titan, Tantal und anderen Metallen.

Zur Lösung dieses Problems sieht die Erfindung ein Verfahren und eine Vorrichtung zur Herstellung eines Pulvers aus Nano-, Mikron- und Submikron-Partikeln von Metallen und/oder von Carbiden der Metalle aus Abfallprodukten und Hartmetallen vor, unter Verwendung von Bakterien, nämlich eines Bakterien-Verbandes mit Acidithiobacillus ferrooxidans, Acidithiobacillus thiooxidans, Leptospirillum ferrooxidans und Ferroplasma acidiphilum; die Einzelheiten des Verfahrens sind im Anspruch 1 angegeben, die Einzelheiten der Vorrichtung im Anspruch 12. Bevorzugte Ausgestaltungen finden sich in den jeweiligen Unteransprüchen.

Der Reaktor (im Folgenden Reaktor-Behälter) wird mit einer bakteriellen Laugenlösung in einem Gewichtsverhältnis zwischen flüssiger Phase und gelösten Festphasen von 0,1 : 1,5 bis 1,0 : 10,0 unter kontinuierlichem Rühren beschickt. Eine biologische Lösung (nachstehend Dispergierflüssigkeit) ist eine wässrige Lösung von Eisensulfat, welche der Lebensraum von autotrophen Thiobakterien ist, insbesondere von Thiobacillus ferrooxidans und Thiobacillus thiooxidans. Die Wirksamkeit der Lösung wird bestimmt durch das Oxidationspotential und die Konzentration von $Fe_3$ sowie durch den pH-Wert ($S\text{-}Fe^{3+} > 4$ g/l, pH < 4). Durch Reduzieren der Eisen-III-Konzentration auf weniger als 4 g/l der Aufschlämmung wird die Dispersionslösung zur Regeneration eingestellt.

Dabei ist allerdings zu beachten, dass eine Beschränkung des Verfahrens auf einen einzigen, engen Temperaturbereich von 20 °C bis 30 °C eine aktive Lebenstätigkeit der verwendeten Bakterien Thiobacillus ferroxidans, Thiobacillus thiooxidans in der Dispersionsflüssigkeit verursachen könnte; ferner könnte das verwendete Trennmittel eine geringe Produktivität aufweisen, woraus eine reduzierte Effizienz des industriellen Produktionsvolumens des Nanopulvers folgt.

Ein Verfahren zur Herstellung der Dispersionslösung mit einem hohen Oxidationspotential gewährleistet eine höhere Periode biokatalytischer Eigenschaften von Bakterien, um ihre Widerstandsfähigkeit gegenüber äußeren Faktoren und die Produktivität von Bakterien für die Biooxidation und deren biochemische Aktivität zu verbessern.

Die Entwicklung einer optimalen Regulierung des technologischen Zyklus der Wiederverwertung von soliden, schweren Wolfram-Legierungen, soll in einem Nanopulver resultieren mit den erforderlichen technischen Spezifikationen, die das Gebiet ihrer weiteren Verwendung (Pulvermetallurgie, Metallpolymere, radioaktive Strahlung absorbierende Materialien und photokatalytischen Materialien) definiert; ein wichtiger Faktor ist dabei die gewünschte, besonders kleine Partikelgröße.

Die Erfindung sieht daher vor, dass die Herstellung von Nano- und Submikron-Pulver in vorzugsweise mit variabler Geschwindigkeit drehenden Reaktor-Behältern erfolgt, deren Drehwinkel gemäß einem vorgegebenen Algorithmus gesteuert wird; in diese Reaktor-Behälter werden Abfallstoffe gelegt und in einer Dispersionsflüssigkeit mit einem hohen Oxidationspotential gelöst. Als Dispersionsflüssigkeit dient eine Schwefelsäurelösung, zusammen mit bioorganischen Komplexen von dreiwertigem Eisen ($Fe^{3+}$) mit einem Exo-Polymer, d.h., mit einem von Bakterien an deren Zellwand außen abgesondertem Polymer. Diese bio-organischen Komplexe (bakterielles Eisen) befinden sich auf einem Substrat aus Aktivkohle oder einer Mischung aus Aktivkohle und Glaukonit-Konzentrat, mit einer verzweigten, porösen Struktur.

Das beschriebene Verfahren zur Nanopulvererzeugung aus Reaktor-Behältern hat seine eigenen, zu optimierenden Parameter. In den Ansprüchen sind wirksame Parameterbereiche angegeben, bei deren Beachtung sich gute Ergebnisse erzielen lassen. Besonders optimale Ergebnisse erzielt die Erfindung bei einem Verfahren zur Herstellung eines Pulvers aus nanoskaligen und/oder submikroskopischen Partikeln von W, $WO_3$, WC, einer Feststofflösung mit Carbiden wie WC-TiC-(TaC), unter Rückführung der Schwerprodukte und Hartmetalle enthaltenden Dispersionsflüssigkeit zu den Reaktor-Behältern unter folgenden Parametern:

Die Zerstörung wird in wenigstens einem Reaktor-Behälter mit variabel einstellbarer Drehgeschwindigkeit durchgeführt, bspw. zwischen 0 und 60 U/min, insbesondere nach einem vorgegebenen Algorithmus, wobei der maximale Winkel $\alpha_{max}$ zwischen 0° und 40° liegt. Die Verstellung des Neigungswinkels erfolgt dabei mit einer Rate in dem Bereich von 0,5 bis 2 kompletten Zyklen pro Minute. Als Dispergierflüssigkeit wird eine Schwefelsäurelösung verwendet mit den Parametern $S\text{-}Fe^{3+}$ = 7 bis 20 g/l und einem pH-Wert von 1,5 bis 2,5, welche bioorganische Komplexe enthält - Eisen von $Fe^{3+}$, unter Zu-

gabe von Bakterienkolonien der Stämme Acidithiobacillus ferrooxidans, Acidithiobacillus thiooxidans, Leptospirillum ferrooxidans und Ferroplasma acidiphilum. Die Gemeinschaft von Bakterien ist immobilisiert auf einem festen, porösen Medium, beispielsweise auf Aktivkohle oder einer Mischung aus Aktivkohle mit Glaukonit, welche die Konzentration von Biomasse deutlich erhöhen kann, sowie biochemisch und/oder oxidativ aktive Bakterien. Das Verhältnis zwischen dem Volumen $V_{n.o}$ der mit schweren und/oder harten Legierungen besetzten Produkte - und dem Volumen $V_{b.r}$ der erhaltenen Dispersions-Flüssigkeit beträgt $V_{b.r}/V_{n.o} = 1 : 2$, und die Temperatur der Lösung mit den bioorganischen Komplexen von Eisen-Dispergiermittel wird auf einem Wert $T = 18$ bis 50 °C eingestellt. Die recycelten Produkte in den rotierenden Destruktoren kippen in die Dispergierflüssigkeit mit den bio-organischen Komplexen mit Eisen $Fe_3$. Sodann werden tribochemische Prozesse aktiviert aufgrund chemomechanischer Bindewirkungen, was den Übergang zu intensivieren Metall-Nanopartikeln in der Lösung führt. Anschließend wurde die Aufschlämmung von Teilchen mit Nano- und/oder Submikron-Größe in der bioorganischen Lösung, die Ionen des Ni-Fe (Ni-Fe-Cu), Co-Fe vom Destruktor entladen, und es erfolgte eine gerichtete Trennung, worauf sich eine Desagglomerierung der gereinigten Nanopartikel durch Ultraschall oder mittels Kugelmühlen anschloss; die Lösung mit Stabilisatoren wurde in einem Vakuumofen getrocknet, bei einer Temperatur von 100 °C bis 120 °C sowie unter einem Vakuum von 1 bis 10 mbar, wobei Ni, Cu und/oder Co ausgefällt wurde, unter Änderung des pH-Wertes. Das technische Ergebnis besteht in der Beschaffung von Nanopulvern mit typischen Abmessungen von 100 bis 400 nm. Falls notwendig, lässt sich Wolframoxid erhalten, indem ein belüfteter Muffelofen bei einer Trocknungstemperatur von 550 °C bis 700 °C mit desagglomeriertem Wolframnanopulver in Lösung ohne Stabilisatoren zum Trocknen beschickt wird, wobei sich aus dem Pulver Wolframoxid $WO_3$ bildet, mit einem monoklinen Gitter und Größen der Nanopartikel zwischen 150 und 500 nm. Die Erfindung kann zur Herstellung von Metalllegierungen aus pulvermetallurgischen Materialien verwendet werden, als nanogroße Füllstoffe für radioaktive Metallopolymere, für Materialien mit einer hohen photokatalytischen Leistung, aufgrund der hohen Härte der Maschinenteile beim Schweißen, Sprühen.

Es folgt eine ausführliche Beschreibung der erfindungsgemäßen Lösung des gestellten Problems anhand der beigefügten Zeichnungen. Hierbei zeigen:

Fig. 1 ein technologisches Schema eines erfindungsgemäßen bio-tribohydrometallurgischen Verfahrens zur Herstellung von Nanopulver unter Wiederverwertung von (Abfall-) Produkten aus schweren und harten Legierungen;

Fig. 2 den Aufbau einer von Anlage zur Herstellung von Schwefelsäure-Lösungen von bio-organischen Komplexen von Eisen $Fe^{3+}$ und Exo-Polymeren, d.h. von durch Bakterien extrazellär gebildeten polymeren Verbindungen;

Fig. 3a den Aufbau eines drehbaren Reaktor-Behälters;

Fig. 3b den Reaktor-Behälter aus Fig. 3a in geneigtem Zustand;

Fig. 4a ein Forschungsergebnis betreffend das bio-tribo-metallurgisch erhaltene Wolfram-Nanopulver, nämlich eine Aufnahme mit einem Rasterelektronenmikroskop in einem Betriebsmodus einer Röntgen-Mikroanalyse von Sekundärelektronen;

Fig. 4b ein Forschungsergebnis betreffend das bio-tribo-metallurgisch erhaltene Wolfram-Nanopulver, nämlich das Ergebnis einer Röntgenbeugung an einem DRON-4-07;

Fig. 5 das Ergebnis von Röntgenbeugungsuntersuchungen an Wolframoxid-Nanopulver mittels eines DRON-4-07.

Fig. 6a ein Forschungsergebnis betreffend das bio-tribo-metallurgisch erhaltene Wolfram-Nanopulver, nämlich eine Aufnahme mit einem Rasterelektronenmikroskop in einem Betriebsmodus einer Röntgen-Mikroanalyse von Sekundärelektronen;

Fig. 6b ein Forschungsergebnis betreffend das bio-tribo-metallurgisch erhaltene Wolfram-Nanopulver, nämlich das Ergebnis einer Röntgenbeugung an einem DRON-4-07.

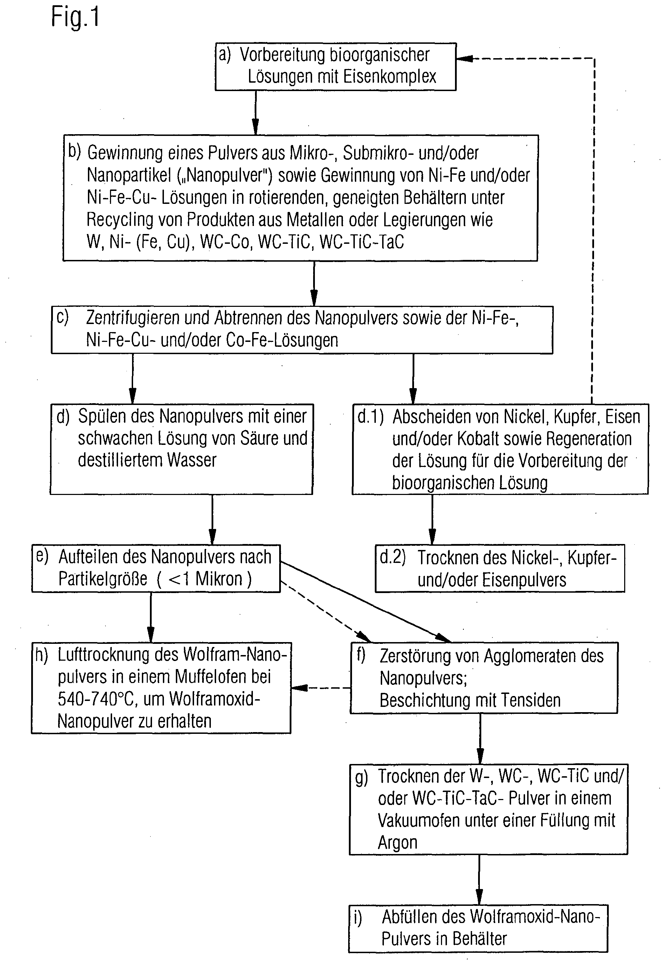

Das vorgeschlagene Verfahren zur bio-tribo-hydrometallurgischen Herstellung eines Pulvers mit Korngrößen im Nano- und/oder Submikron-Bereich aus Abfallprodukten mit Schwer- und/oder Hartmetall wird in mehreren Stufen durchgeführt, vgl. Fig. 1:

a) Herstellung von Schwefelsäurelösungen mit bio-organischen Komplexen ($Fe^{3+}$)

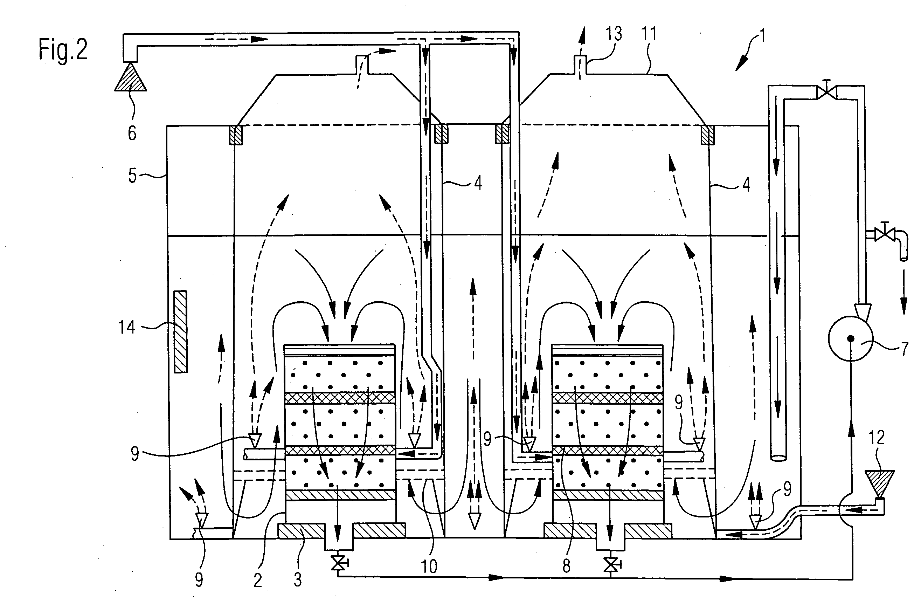

Eine Anlage 1 zur Vorbereitung bioorganischer Lösungen mit Eisenkomplexen zeigt Fig. 2. Man erkennt Behälter 2, die innerhalb eines Tanks 5 zusammen mit einem Substrat fixiert sind.

Als Substrat wird ein Aktivkohlegranulat mit einer Korngröße von 2,4 mm bis 4,8 mm und Glaukonit-Konzentrat (Glaukonit-Gehalt ca. 99 %) verwendet.

Die festen Medien haben eine poröse Struktur entwickelt, wodurch die Konzentration der Biomasse erheblich gesteigert werden kann, und infolgedessen wird die biochemische Aktivität der Bakterien erhöht wie auch die oxidative Aktivität der Bakterien mit zunehmendem Alter der Mikroorganismen. Zusätzlich ist Glaukonit als Quelle von Spurenelementen, insbesondere von K, P und Mg, zur Aktivierung der bakteriellen Zellen erforderlich. Im Rahmen der Biomasse werden die Bakterien Acidithiobacillus ferrooxidans, Acidithiobacillus thiooxidans, Leptospirillum ferrooxidans und Ferroplasmy acidiphilum verwendet. Der Behälter wird mit einer Lösung von zweiwertigem Eisensulfat $FeSO_4 \cdot 7 H_2O$ mit einer Konzentration der Ionen $Fe^{2+}$ = 12 gefüllt bis zu einem Anteil 25 g/l, und mit einem pH-Wert von 1,5 bis 2,5, wobei der pH-Wert mit Schwefelsäure eingestellt wird.

Eine Lösung von Eisen wird durch die Pumpe 7 in der gesättigten Luft der Konzentrations-Mischer 4 über die Behälter 2 gepumpt. Dabei erfolgt die Oxidation von $Fe^{2+}$ zu $Fe^{3+}$, sowie die Bildung von komplexen assoziierten Verbindungen (bakterielles Eisen), die bio-organische Komplexe sind - Eisen $Fe^{3+}$ mit Exo-Polymer - polymeren Substanzen also, die von Bakterien produziert werden. Das Volumen der durch den festen Träger 20 pro Stunde gepumpten Lösung sollte im Bereich des 3-fachen Volumens liegen. Eine Sättigung der Lösung mit Luft erfolgt über Düsen 9 in den Naben-Mischer 4 und dem Tank 5. Die Menge an Luft durch den Kompressor 6 und 12 zugeführt wird, 1 bis 1,5 Volumen der Gesamtlösung.

Um die Oxidation von Eisen zu beschleunigen sowie die Wachstumsrate der Mikroorganismen in der Lösung, ist vorteilhaft, Ammonium-Trinatriumphospat $(NH_4)3 PO_4 \cdot 3 H_2O$ hinzuzufügen. Der für Bakterien optimale Temperaturbereich von 20 bis 35 °C wird mittels eines Thermostats 15 aufrechterhalten. Das bakterielle Ferrieisen (pH = 1,5 bis 2,5) wird durch eine Pumpe entleert, wenn die Konzentration der bioorganischen Komplexe $Fe^{3+}$ 7 bis 20 g/l beträgt (20 g/l = 7 + SFE3).

b) Herstellung von nanoskaligem (Submikron-) Pulver sowie der Lösungen von Ni-Fe, Ni-Fe-Cu, und/oder Co-Fe in rotierenden Reaktor-Behältern beim Recycling von Produkten aus den Legierungen W-Ni (Fe, Cu), WC-Co, WC-TiC-(TaC)-Co

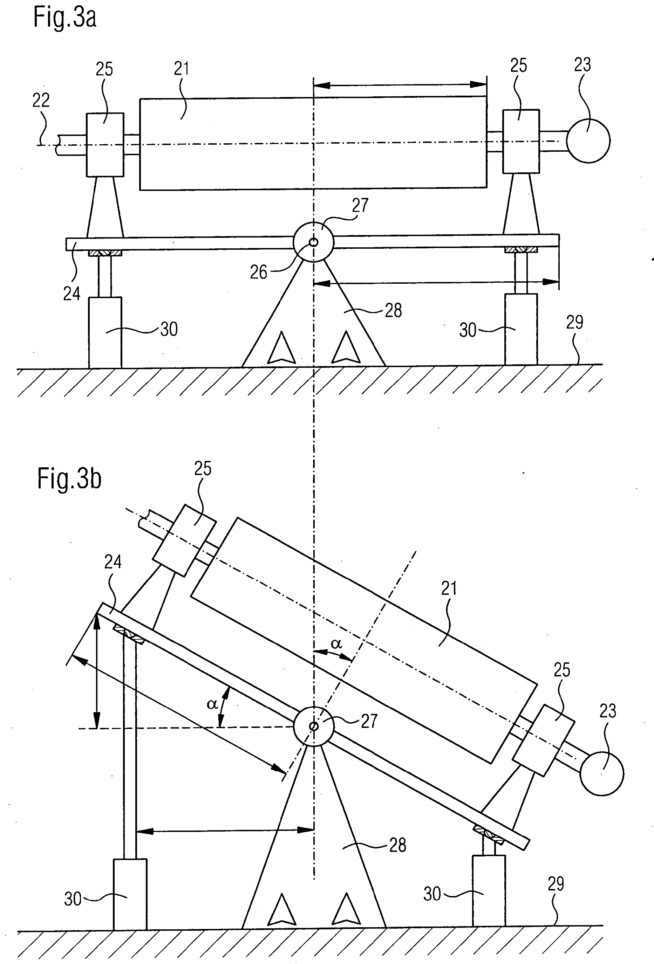

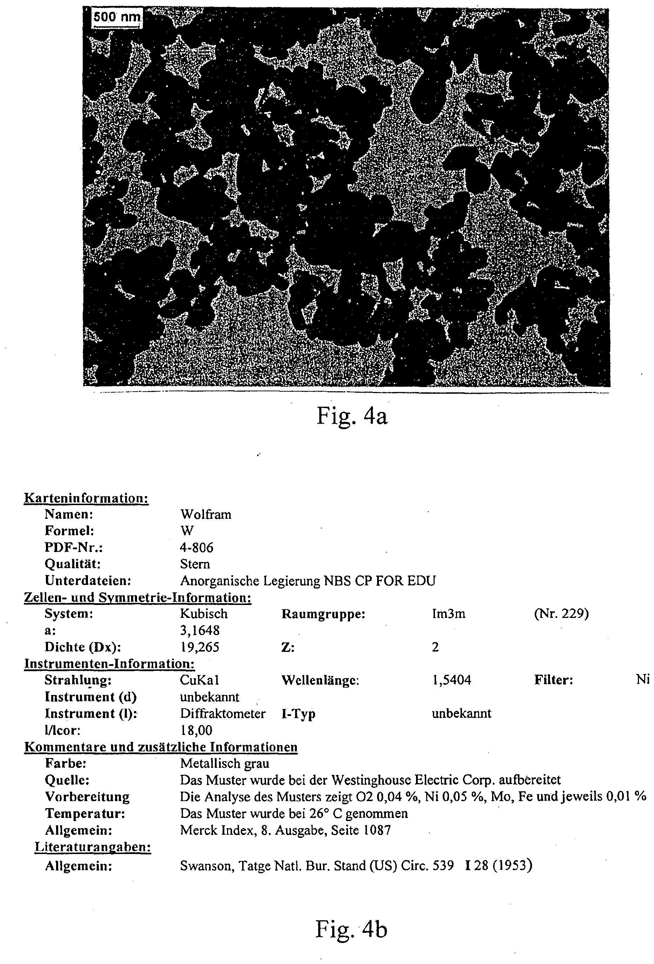

Das Verfahren zur Gewinnung eines Pulvers findet in rotierenden Reaktor-Behältern 21 statt, die in den Fig. 3 und 4 dargestellt sind. Ein solcher Reaktor-Behälter 21 ist als Sechskantprisma ausgebildet, dessen Mantelfläche seine Längsachse 22 umgibt.

Von einem Elektromotor 23 kann ein solcher Reaktor-Behälter 21 um seine Längsachse 22 drehend angetrieben werden, wobei die Drehgeschwindigkeit in einem Bereich von 0 bis 60 U/min eingestellt und/oder geregelt werden kann.

Ferner kann der Neigungswinkel a eines solchen Reaktor-Behälters 21 variiert werden durch Verstellen des Neigungswinkels a einer Plattform 24, auf welcher der Reaktor-Behälter 21 mittels Lagerbaugruppen 25 starr befestigt ist. Dies wird dadurch ermöglicht, indem die Plattform 24 um eine Achse 26 drehbar gelagert ist, welche sich in einem etwa mittigen Bereich der Plattform 24 quer zu der Längsachse 22 des Reaktor-Behälters 21 erstreckt. Zu diesem Zweck ist wenigstens ein Lagerelement 27 über einen Fuß 28 an einem Fundament 29 abgestützt.

Die aktive Einstellung des Neigungswinkels a erfolgt vorzugsweise mittels eines oder mehrerer Hydraulikzylinder 30, welche etwa im Bereich unterhalb (je) eines Endes des Reaktor-Behälters 21 zwischen der Plattform 24 und dem Fundament 29 angeordnet sind. Der maximale Betrag des Neigungswinkels $\alpha_{max}$ ist auf höchstens 40° begrenzt:

$$-\alpha_{max} \leq \alpha \leq +\alpha_{max},$$

bzw.:

$$-40° \leq \alpha \leq +40°.$$

Der Reaktor-Behälter 21 kann in zwei unterschiedlichen Modi betrieben werden - (i) Betrieb mit einem stationären Neigungswinkel $\alpha$ einerseits, sowie (ii) zyklische Veränderungen des Neigungswinkels $\alpha$ andererseits. Bei der zyklischen Veränderung des Neigungswinkels $\alpha$ gibt ein Algorithmus bzw. ein Programm den Winkel $\alpha_{max}$ in einem Bereich von 0° bis 40° vor, und auch einen Wert für die in einem Bereich von 0,5 bis 2 vollständigen Zyklen der Veränderung des Neigungswinkels pro Minute einstellbare Änderungsrate des Neigungswinkels a. Ein voller Zyklus des Programms umfasst das einmalige Kippen des Reaktor-Behälters 21 in jeweils beide Richtungen, also über die Neigungswinkel $(+\alpha_{max})$ - 0° - $(-\alpha_{max})$ - 0° - $(+\alpha_{max})$.

Neben der stationären Betriebsweise ohne Kippen des Reaktor-Behälters 21 und der zyklischen Betriebsweise mit zyklischem Kippen des Reaktor-Behälters 21 gibt es grundsätzlich noch die unterbrochene, zyklische Betriebsweise, wobei der Reaktor-Behälter 21 nur zeitweise gekippt wird und in den dazwischen liegenden Zeitintervallen nicht. Man kann sich dies so vorstellen, dass während des Betriebs wiederholt zwischen der kontinuierlichen Betriebsweise und der zyklischen Betriebsweise umgeschalten wird. Außerdem können auch während der Prozessdauer die Parameter des zyklischen Betriebs variiert werden.

Die Regelung der Drehzahl des Reaktor-Behälters 21 um dessen Längsachse 22 wird anhand eines Algorithmus durchgeführt, welcher von der Änderungsrate des Neigungswinkels $\alpha$ unabhängig ist, um ein individuelles Programm für das Recycling von Produkten implementieren zu können.

Der Reaktor-Behälter 21 wird mit Produkten aus wolframhaltigem Schrott so weit beladen, dass wenigstens ¼ des Volumens des Reaktor-Behälters 21 frei bleibt, vorzugsweise mehr als 1/3 des Volumens des Reaktor-Behälters 21.

Die maximale Erstreckung der eingefüllten Produkte sollte einen Wert von 4 cm nicht überschreiten; kleinere Produkte werden bevorzugt, beispielsweise mit einer maximalen Erstreckung von 1 cm bis 4 cm, oder gar von höchstens 1 cm. Zu große Einzelteile sollten zuvor auf die angegebene Größe vorgeschliffen werden. Dann wird die Dispergierflüssigkeit in den Reaktor-Behälter 21 gegossen - Schwefelsäurelösung mit bio-organischen Komplexen mit $Fe^{3+}$, sowie mit einem Parameter SFE3 = 7 bis 20 g/l und einem pH-Wert von 1,5 - 2,5. Für das Verhältnis des Volumens der Dispersionsflüssigkeit $V_{b.r}$ zu dem von den (Abfall-) Produkten aus schweren und harten Legierungen besetzten Volumen $V_{n.o}$, sollte gelten:

$$1 : 4 \leq V_{b.r} / V_{n.o} \leq 1 : 0,5,$$

vorzugsweise

$$1 : 3 \leq V_{b.r} / V_{n.o} \leq 1 : 1,$$

insbesondere

$$1 : 2,5 \leq V_{b.r} / V_{n.o} \leq 1 : 1,5,$$

im Idealfall

$$V_{b.r} / V_{n.o} = 1 : 2.$$

Im Rahmen der Interaktion der Dispersion von Produkten mit einem hohen Redoxpotential in der Flüssigkeit spielen sich in einem rotierenden Reaktor-Behälter 21 intensive tribochemische Prozesse ab, was zur Bildung von hochgradig dispergierten Suspensionen von Nano- und Submikronteilchen W, WC WC-Tic (TaC) in der Lösung führt. Der Mechanismus der Bildung von Suspensionen erfolgt durch zwei Effekte - hämo-mechanischer Effekt und Rehbinder-Effekt.

Während der Herstellung werden sich die schweren Wolfram-Legierungen mit den festen Bindemetallen Ni, Cu, Co-Kristalliten vermischen. Beim Kontakt mit den in dem Dispergierungsfluid enthaltenen bio-organischen $Fe^{3+}$-Komplexen wird durch den hämo-mechanischen Effekt eine intensive Zerstörung der Oberfläche der Produkte verursacht sowie der Übergang von Ni-, Cu- und/oder Co- Ionen in die Lösung. Als Ergebnis wird die Legierungsoberfläche mit einer Vielzahl von Defekten in der Kristallstruktur übersät. Die Geschwindigkeit des Prozesses hängt von der Konzentration und der Temperatur der Standard-$Fe^{3+}$-Lösung mit den bio-organischen Eisen-Komplexen ab, für Werte von T = 18 °C bis 50 °C.

Durch Ändern des Neigungswinkels a und der Reibfläche der Produkte relativ zueinander während der Rotation des Reaktor-Behälters 21 ergeben sich chaotische Kollisionsfragmente der Produkte, was infolge des Rehbinder-Effektes zur Bildung einer Schicht auf der kontinuierlich zerstörten Oberfläche führt.

Die Unterbrechung dieser Schicht führt zu einem Übergang der Nano- und Submikronteilchen aus W, WC, WC-TiC (TaC) in die Lösung. Die Intensität des Prozesses wird bestimmt durch die Drehgeschwindigkeit des Reaktor-Behälters, die Betriebsartenwahl, den Steueralgorithmus und die Änderungsrate des Neigungswinkels a pro Minute.

Als Ergebnis bildete sich eine Aufschlämmungslösung von Teilchen, die gleichzeitig, ähnlich wie ein Schleifmittel, eine Intensivierung der Bruchfläche sowie die Beförderung von Teilchen von der Oberfläche der Legierung in die Lösung fördert.

c) Trennung der Nano- und Submikron-Pulver und der Lösung von Ni-Fe (Ni-Fe-Cu), und/oder Co-Fe

Nach dem Schritt b) wird die Suspension aus den Nano- und Submikronteilchen in einer bio-organische Lösung, die Ionen des Ni-Fe (Ni-FE-Cu), Co-Fe enthält, von dem Reaktor-Behälter 21 entnommen und einer Zentrifuge zugeleitet. Während des Zentrifugierens erfolgt eine Trennung der ausgefällten Feststoff-Fraktion von einer flüssigen Fraktion in Form der Ni-Fe-, Ni-Fe-Cu-, und/oder Co-Fe- Lösung.

d,d1,d2) Spülen des Nano-Pulvers sowie Ausscheiden von Nickel, Kupfer, Eisen und/oder Kobalt sowie Regeneration der $Fe^{2+}$-Lösung zur Herstellung einer $Fe^{3+}$-Lösung mit bio-organischen Komplexen

Nach dem Zentrifugieren des Nano- und Submikron-Pulvers wird dieses mit einer schwachen Lösung aus Schwefelsäure und destilliertem Wasser gewaschen, um Ni-, Cu-, Co-, und/oder Fe-Reste von der Oberfläche zu entfernen. Das Kriterium für die Beendigung des Waschens ist erfüllt, wenn die gewünschte chemische Reinheit des Pulvers von beispielsweise 99,95 % erreicht ist. Durch die Abscheidung von Ni, Cu, Co und/oder Fe verändert sich der pH-Wert der Lösung. Waschlösungen können in einem anschließenden Schritt a) wieder verwendet werden.

Die Pellets aus Ni, Cu, Co und/oder Fe werden filtriert und in einem Muffelofen getrocknet. Der Rest der $Fe^{2+}$-Lösung kann für eine Regeneration der $FeSO_4$-Lösung verwendet werden sowie für die anschließende Verwendung in einem folgenden Schritt a).

e) Trennen der Nanopartikel

Das im Schritt b) erhaltene Nanopulver hat eine Partikelgrößenverteilung von 5 nm bis 1 μm. Die mittlere Ggröße eines Nanopartikels liegt zwischen 100 nm und 400 nm. Basierend auf den Ergebnissen der Rastertunnelmikroskopie ergibt sich, dass mindestens 80 % der Körner der Probe des im Schritt b) erhaltenen Nanopulvers eine Teilchengröße von weniger als 500 nm haben.

Falls erforderlich, erfolgt die Aufspaltung der Nanofraktionen im Rahmen einer Tangentialströmungsfiltration mit keramischen Rohrmodulen (Cross-Flow-Technik), oder auch mittels Systemen für die asymmetrische Fluss-Fraktionierung oder Zentrifugalfraktionierung.

Die Größenverteilung des resultierenden Nanopulvers ist jedoch optimal für die Pulvermetallurgie, für Metallpolymere und für Materialien mit photokatalytischen Eigenschaften.

f) Zerstörung von Agglomeraten und von Tensid-Schichten aus Ölsäure, Palmitinsäure, Imidazolin Oleylalkohol

Nach dem Schritt d) (oder, falls erforderlich, nach dem Schritt (e)) wird zur Desagglomerierung die Nanodispersion in einer Lösung mit Stabilisatoren (Tensiden) einer Beschallung mit Ultraschall ausgesetzt oder in Kugelmühlen gemahlen.

Wenn als Endprodukt Wolframoxid $WO_3$ gewünscht ist, so ist die Verwendung von Stabilisatoren nicht erforderlich.

g) Trocknen des Pulvers aus W, WC, WC-TiC-(TaC) in einem Vakuumofen und Aufbewahrung unter einem inerten Gas wie Argon

Das mit Stabilisatoren beschichtete Nanopulver wird in eine oder mehrere Schalen gefüllt und in einem Vakuumofen getrocknet. Das Trocknen erfolgt im Vakuum, um die Feuchtigkeit und Konzentration von Gasen, insbesondere Sauerstoff, in dem Nanopulver zu reduzieren. Die Trocknung wird bei einem Restdruck von etwa 10 mbar bei einer Temperatur von 100 °C bis 120 °C durchgeführt. Nach Abschluss der Trocknung wird/werden die Schale(n) mit einem inerten Gas gefüllt, vorzugsweise mit Argon, welches den Kontakt des Pulvers mit der atmosphärischen Luft verhindert.

g) Gewinnung von Nanopulver als Wolframoxid $WO_3$

Wenn als Endprodukt Wolframoxid gewonnen werden soll, so wird nach der Trocknung im Schritt f) Luft zu dem Wolframpulver in den Muffelofen geleitet, bei einer Trocknungstemperatur von 550 °C bis 700 °C. Dadurch bildet sich aus dem Pulver Wolframoxid $WO_3$ mit einem monoklinen Gitter, was eine hohe photokalytische Leistung darstellt.

Damit ist die der Erfindung zugrunde liegende Aufgabe gelöst.

Die biologische Wirksamkeit von tribo-hydrometallurgischen Verfahren für die Herstellung von Nanopulvern unter Wiederverwertung von (Abfall-) Produkten mit/und festen Wolframschwerlegierungen W-Ni- (Fe, Cu), WC-Co, WC-TiC-(TaC)-Co wird durch die folgenden Beispiele veranschaulicht.

Beispiel 1:

Die verwendeten Rohstoffe waren Abfallprodukte aus Legierungen von W-Ni und W-Ni-Fe, in Form von Wolfram-Metall-Schrott.

Die durchschnittliche Menge an Abfallprodukten hatte eine Größe von 1 bis 2 cm. Ein Reaktor-Behälter 21 wurde mit 50 kg Abfall beladen. Dann wurde der Reaktor-Behälter 21 überflutet mit einer Dispergierflüssigkeit in Form einer Sulfat-Lösung mit bio-organischen Komplexen aus $Fe^{3+}$ und Exo-Polymeren, d.h. von Bakterien extrazellulär produzierten polymeren Substanzen. Die in dem vorangehenden Schritt a) verwendete Lösung hatte die Parameter $S-Fe^{3+}$ = 15 g/l und pH = 2. Das Volumen der Flüssigkeit war 8 Liter. Das Volumen der verarbeiteten Abfälle war zusammen mit einer Lösung aus etwa 11 Liter hergestellt.

Als Betriebsmodus für den verwendeten Reaktor-Behälter 21 wurde die kontinuierliche Betriebsweise ausgewählt. Die Drehgeschwindigkeit des Reaktor-Behälters lag bei 21 U/min bis 25 U/min, der maximale Neigungswinkel $\alpha_{max}$ lag bei 25°, die Rate der Änderung des Neigungswinkels $\alpha$ bei 1,5 vollständigen Zyklen pro Minute, und die Temperatur der Lösung wurde bei 30 °C bis 32 °C gehalten.

Einmal in zwei Tagen wurde kontrolliert, ob der pH-Wert bei pH = 2 lag, und ggf. entsprechend eingestellt; nach 5 - 6 Tagen erhielt man eine gemischte Aufschlämmung, bestehend aus Nano- und Submikron-Partikel in einer bio-organische Lösung. Dann, nach Schritt c) und d), wurde das Wolfram-Nanopulver getrennt und in einer Lösung aus Schwefelsäure und destilliertem Wasser gewaschen. Anschließend wurde das Wolfram-Nanopulver getrocknet in einem Vakuumofen bei einer durch ein Programm vorgegebenen maximalen Temperatur von 100 °C sowie unter einem Vakuum von 5 mbar.

Ein Teil des resultierenden Nanopulvers wurde bei einer Temperatur von 620 °C nach dem Trocknen im Muffelofen der Luft ausgesetzt. Nach dem Trocknen des Nanopulvers erhielt man Wolframoxid $WO_3$.

Die Ausbeute des Nanopulvers W lag bei 47 % des ursprünglichen Gewichts des geladenen Metalls. Bei diesem Beispiel ergab sich eine gesamte Nanopulver-Ausbeute von 23,5 kg / Monat.

Zu Studienzwecken wurde die Teilchengröße bestimmt anhand einer rasterelektronenmikroskopischen Aufnahme mittels eines "REMMA 102"-Mikroskops sowie mit einer Röntgen-Mikroanalyse. Dabei lag die maximale Auflösung bei 10 nm, die Beschleunigungsspannung an der Elektronensonde bei 20 kV, der Sondenstrom bei 3 nA, und die Belichtungszeit bei 200 s.

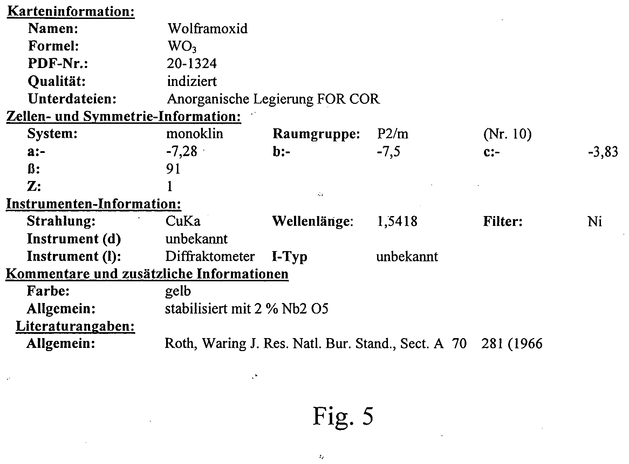

Die Struktur des Nanopulvers wurde mittels eines Röntgendiffraktometers DRON-4-07 untersucht. Bei der Aufnahme wurde CuKa-Strahlung verwendet (Wellenlänge 0,154 nm), mit einem Schwerpunkt der Bragg-Brentano-Anordnung von ?-2? (Braggwinkel 2?). Die Spannungs- und Stromwerte an der Röntgenstrahlröhre lagen bei 40 kV und 20 mA. Die Identifizierung der kristallinen Phasen wurde mit Hilfe des Software-Paketes "Crystallographica Search-Match" (Oxford Cryosysytems) durchgeführt, in der erweiterten Form. Daraus ergab sich, dass die kristallinen $WO_3$-Nanopartikel eine monokline Gitterstruktur haben (Fig. 5).

Beispiel 2:

Als Rohstoff wurde eine Produktlegierung WC-Co (Schneidmesser) verwendet. Die Durchschnittsgröße der verwendeten Abfall-Partikel lag bei 1 cm bis 1,5 cm.

Der Reaktor-Behälter 21 wurde mit einer Menge von 70 kg Abfall-Produkten in Form von Wolfram-Metall-Schrott beladen. Daraufhin wurde der Reaktor-Behälter 21 mit einer Dispergierflüssigkeit überflutet, nämlich mit einer Sulfat-Lösung, die bio-organische Komplexe aus $Fe^{3+}$ und Exopolymeren, d.h., von Bakterien extrazellulär produzierten, polymeren Substanzen, enthielt. Die dabei verwendete Dispergierflüssigkeit hatte eine $S$-$Fe^{3+}$-Konzentration von 12 g/l und einen pH-Wert von 1,5.

Die Ergebnisse zeigten, dass bei dem Wolfram-Nanopulver die grundlegende Dimension der Nanopartikel von 100 nm bis 300 nm reichte, siehe Fig. 4a, und dass das Kristallgitter kubisch war, siehe Fig. 4b.

Im Hinblick auf die unregelmäßig geformten und agglomerierten Pulverpartikel wurde die Betriebsart des Reaktorbehälters 21 programmiert auf die abwechselnde Wiederholung zweier Zyklen:
In der ersten Hälfte einer betrachten Periode - 1 Stunde - lag die Drehzahl der Rotation des Reaktor-Behälters 21 bei 20 U/min, der maximale Neigungswinkel $\alpha_{max}$ des Reaktors 21 lag bei $\alpha_{max} = 30°$, und die Periode der Winkeländerung für einen kompletten Zyklus der Änderung des Winkels $\alpha$ lag bei einer Minute.

In der zweiten Hälfte des betrachteten Zeitraums - ebenfalls 1 Stunde - lag die Drehzahl der Rotation des Reaktor-Behälters 21 bei 40 U/min, der maximale Neigungswinkel $\alpha_{max}$ des Reaktors 21 lag bei $\alpha_{max} = 20°$, und die Periode der Winkeländerung für einen kompletten Zyklus der Änderung des Winkels $\alpha$ lag bei 2 kompletten Zyklen pro Minute.

Die Temperatur der Lösung wurde auf 35 °C gehalten.

Einmal in zwei Tagen wurde der pH-Wert überwacht und ggf. eingegriffen, um einen pH-Wert von 1,5 zu erhalten; nach 5 bis 6 Tagen erhielt man eine gemischte Aufschlämmung aus Nano- und Submikronteilchen in einer bio-organischen Lösung. Dann, nach Schritt c) und d), wurde das Nanopulver getrennt und in einer Lösung aus Schwefelsäure und destilliertem Wasser gewaschen. Anschließend wurde das WC-Nanopulver in einem programmgesteuerten Vakuumofen getrocknet, wobei die maximale Temperatur bei 120 ° C lag, und das Vakuum bei 10 mbar.

Die Ausbeute des WC-Nanopulvers lag bei 50 % des ursprünglichen Gewichts des geladenen Metalls. Bei diesem Beispiel ergab sich eine gesamte Nanopulver-Ausbeute von 35 kg / Monat.

Zu Studienzwecken wurde die Teilchengröße bestimmt anhand einer rasterelektronenmikroskopischen Aufnahme mittels eines "REMMA 102"-Mikroskops sowie mit einer Röntgen-Mikroanalyse. Dabei lag die maximale Auflösung bei 10 nm, die Beschleunigungsspannung an der Elektronensonde bei 20 kV, der Sondenstrom bei 3 nA, und die Belichtungszeit bei 200 s.

Die Struktur des Nanopulvers wurde mittels eines Röntgendiffraktometers DRON-4-07 untersucht. Bei der Aufnahme wurde CuKa-Strahlung verwendet (Wellenlänge 0,154 nm), mit einer Bragg-Brentano-Anordnung und einem Braggwinkel 2?. Die Spannungs- und Stromwerte an der Röntgenstrahlröhre lagen bei 40 kV und 20 mA. Die Identifizierung der kristallinen Phasen wurde mit Hilfe des Software-Paketes "Crystallographica Search-Match" (Oxford Cryosysytems) durchgeführt.

Daraus ergab sich, dass die kristallinen $WO_3$-Nanopartikel eine monokline Gitterstruktur haben (Fig. 5).

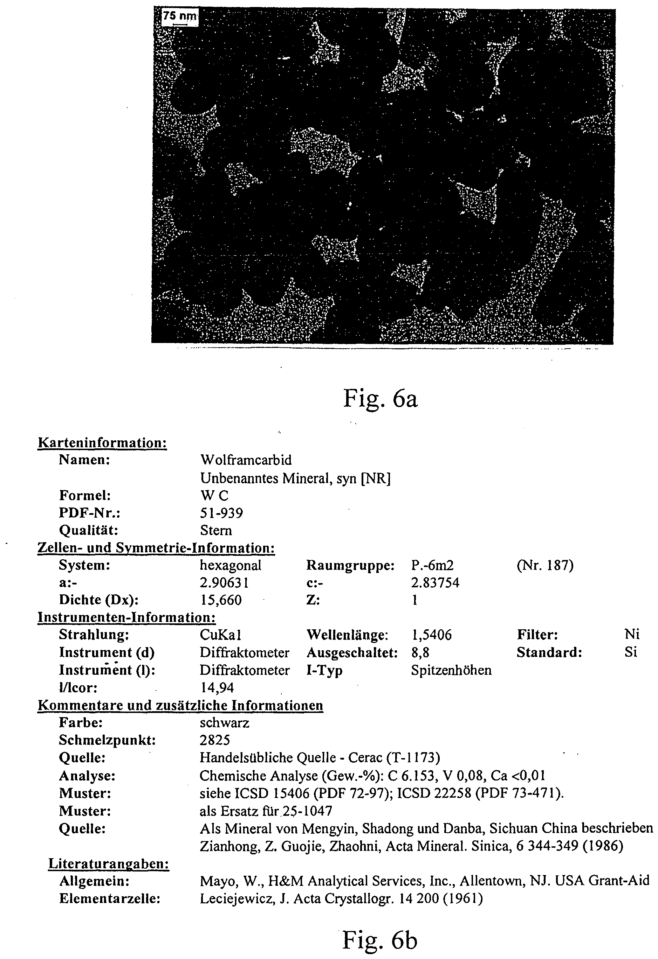

Die Ergebnisse zeigten, dass die Korngröße der meisten Nanopartikel des WC-Nanopulvers zwischen 150 nm und 300 nm lag (Fig. 6a), und dass das Kristallgitter der WC-Nanopartikel hexagonal ist (Fig. 6b). Agglomerierte Pulverteilchen haben eine unregelmäßige, im Wesentlichen längliche Form.

Die chemische Analyse des Pulvers zeigte, dass der Kohlenstoffgehalt bei C = 6,15 % lag.

Bezugszeichenliste

| 1 | Anlage |
|---|---|
| 2 | Behälter |
| 3 | Boden |
| 4 | Mischer |
| 5 | Tank |
| 6 | Kompressor |
| 7 | Pumpe |
| 8 | Substrat |
| 9 | Düse |
| 10 | Strömung |
| 11 | Deckel |
| 12 | Kompressor |
| 13 | Auslass |
| 14 | Thermostat |

21 Reaktor-Behälter
22 Längsachse
23 Elektromotor
24 Plattform
25 Lagerbaugruppe
26 Achse
27 Lagerelement
28 Fuß
29 Fundament
30 Hydraulikzylinder

**Patentansprüche**

1. Verfahren zur Gewinnung eines Pulvers aus Partikeln von Wolfram oder Wolframverbindungen mit einer Partikelgröße im Nano-, Mikron- oder Submikronbereich, insbesondere von W, $WO_3$, WC oder WC-TiC, WC-TaC oder WC-TiC-TaC, unter Recyceln von Produkten aus Schwermetall- oder Hartmetall-Legierungen in wenigstens einem eine Dispersionsflüssigkeit enthaltenden Reaktor-Behälter (21), **dadurch gekennzeichnet, dass**

   a) der Abbau der Produkte bei einer Drehung des Reaktor-Behälters (21) mit einer in einem Bereich zwischen 0 und 90 U/min nach einem vorgegebenen Algorithmus kontrolliert einstellbaren Drehzahl durchgeführt wird, wobei im Rahmen des vorgegebenen Algorithmus der maximale Neigungswinkel ($\alpha_{max}$) des Reaktor-Behälters (21) zwischen 0° und 40° liegt und die Änderungsrate des Neigungswinkels (a) in einem Bereich zwischen 0,5 und 2 kompletten Zyklen pro Minute liegt, wobei ein voller Zyklus des Programms das einmalige Kippen des Reaktor-Behälters (21) in jeweils beide Richtungen umfasst, also über die Neigungswinkel $(+\alpha_{max})$ - 0° - $(-\alpha_{max})$ - 0° - $(+\alpha_{max})$;
   b) wobei als Dispergierflüssigkeit Schwefelsäure mit gelöstem Eisen verwendet wird mit S-$Fe^{3+}$ = 5 bis 30 g/l und einem pH-Wert von 1 bis 3, und welche bioorganische Komplexe aus $Fe^{3+}$ und Exo-Polymeren enthält;
   c) mit einem zugeordneten Bakterien-Verband von Acidithiobacillus ferrooxidans, Acidithiobacillus thiooxidans, Leptospirillum ferrooxidans und Ferroplasma acidiphilum;
   d) wobei das Verhältnis zwischen dem Volumen $V_{n.o}$ der Produkte mit Schwer- und/oder Hartmetall-Legierungen zu dem Volumen $V_{b.r}$ der Dispersionsflüssigkeit bei 1 : 4 < $V_{b.r}$ / $V_{n.o}$ ≤ 1 : 0,5 liegt;
   e) wobei die Temperatur T der Lösung der bioorganischen Eisenkomplexe in dem Dispergiermittel in einem Bereich von 10 °C bis 60 °C gehalten wird;
   f) wobei anschließend von der aus Nano-, Mikron- und/oder Submikron-Partikeln in der bioorganischen Lösung bestehenden Suspension Ionen von Ni-Fe, Ni-Fe-Cu und/oder Co-Fe abgegeben und abgetrennt werden, wobei sich durch die Abtrennung von Ni, Cu und/oder Co der pH-Wert ändert;
   g) wobei das Nanopulver nach der Reinigung einer Desagglomeration mit Kugelmühlen oder mit Ultraschall unterzogen wird, ggf. mit einer Stabilisatorlösung;
   h) und anschließend in einem Vakuumofen bei einer Temperatur von 90 °C bis 130 °C sowie unter einem Vakuum von 1 mbar bis 50 mbar getrocknet wird.

2. Verfahren nach Anspruch 1, **dadurch gekennzeichnet, dass** das Wolfram-Nanopulver nach der Desagglomeration mittels Ultraschall in einer Lösung ohne Stabilisatoren zum Trocknen in einen belüfteten Muffelofen bei einer Temperatur von 550 °C bis 700 °C geladen wird, wobei sich das NanoPulver zu $WO_3$ oxidiert, mit einem monoklinen Gitter und einer Größe der Nanopartikel von 150 nm bis 500 nm.

3. Verfahren nach Anspruch 1 oder 2, **dadurch gekennzeichnet, dass** der Bakterien-Verband von Acidithiobacillus ferrooxidans, Acidithiobacillus thiooxidans, Leptospirillum ferrooxidans und Ferroplasma acidiphilum; auf einem festen porösen Träger, insbesondere auf Aktivkohle oder auf Aktivkohle mit Glaukonit, immobilisiert ist.

4. Verfahren nach einem der Ansprüche 1 bis 3, **dadurch gekennzeichnet, dass** das Exo-Polymer von den Bakterien extrazellulär außen auf der Zellwand produziert wird.

5. Verfahren nach einem der vorhergehenden Ansprüchen, **dadurch gekennzeichnet, dass** im Schritt a) die Drehung des Reaktor-Behälters (21) mit einer in einem Bereich zwischen 0 und 60 U/min durchgeführt wird.

6. Verfahren nach einem der vorhergehenden Ansprüchen, **dadurch gekennzeichnet, dass** die Dispergierflüssigkeit eine Konzentration S-$Fe^{3+}$ = 7 bis 20 g/l aufweist.

7. Verfahren nach einem der vorhergehenden Ansprüchen, **dadurch gekennzeichnet, dass** die Dispergierflüssigkeit einen pH-Wert von 1,5 bis 2,5 aufweist.

8. Verfahren nach einem der vorhergehenden Ansprüchen, **dadurch gekennzeichnet, dass** das Verhältnis zwischen dem Volumen $V_{n.o}$ der Produkte mit Schwer- und/oder Hartmetall-Legierungen zu dem Volumen $V_{b.r}$ der Dispersionsflüssigkeit bei $1 : 3 \leq V_{b.r} / V_{n.o} \leq 1 : 1$ liegt, vorzugsweise in einem Bereich $1 : 2,5 \leq V_{b.r} / V_{n.o} \leq 1 : 1,5$, insbesondere bei $V_{b.r} / V_{n.o} \leq 1 : 2$.

9. Verfahren nach einem der vorhergehenden Ansprüchen, **dadurch gekennzeichnet, dass** die Temperatur T der Lösung der bioorganischen Eisenkomplexe in dem Dispergiermittel in einem Bereich von 18 °C bis 50 °C gehalten wird.

10. Verfahren nach einem der vorhergehenden Ansprüchen, **dadurch gekennzeichnet, dass** im Schritt h) die Temperatur des Vakuumofens in einem Bereich von 100 °C bis 120 °C gehalten wird.

11. Verfahren nach einem der vorhergehenden Ansprüchen, **dadurch gekennzeichnet, dass** im Schritt h) das Vakuum in dem Vakuumofen in einem Bereich von 8 mbar bis 20 mbar gehalten wird, insbesondere in einem Bereich von 5 mbar bis 10 mbar.

12. Vorrichtung zur Gewinnung eines Pulvers aus Partikeln von Wolfram oder Wolframverbindungen mit einer Partikelgröße im Nano-, Mikron- oder Submikronbereich, insbesondere von W, $WO_3$, WC, WC-TiC, WC-TaC oder WC-TiC-TaC, unter Recyceln von Produkten aus Schwermetall- oder Hartmetall-Legierungen, mit wenigstens einem eine Dispersionsflüssigkeit enthaltenden, beispielsweise rotationssymmetrischen, vorzugsweise länglichen oder langgestreckten Reaktor-Behälter (21) zum Abbau der Produkte, insbesondere nach dem Verfahren gemäß einem der vorhergehenden Ansprüche, **gekennzeichnet durch**

a) eine Einrichtung, um den Reaktor-Behälter (21) in Rotation um eine Drehachse, vorzugsweise um seine Längsachse, zu versetzen, bei einer zulässigen Umdrehungszahl des Reaktor-Behälters (21) wenigstens in einem Bereich zwischen 0 und 90 U/min;
b) eine Einrichtung, um den maximalen Neigungswinkel ($\alpha_{max}$) der Drehachse des Reaktor-Behälters (21), insbesondere dessen Längsachse, zwischen 0° und 40° zu variieren;
c) eine Einrichtung zur Steuerung der Umdrehungszahl des Reaktor-Behälters (21) wenigstens in einem Bereich zwischen 0 und 90 U/min;
d) eine Einrichtung zur Steuerung und zyklischen Veränderung des Neigungswinkels (a) der Drehachse oder der Längsachse des Reaktor-Behälters (21) derart, dass die Änderungsrate des Neigungswinkels (a) in einem Bereich zwischen 0,5 und 2 komplette Zyklen pro Minute liegt, wobei ein voller Zyklus des Programms das einmalige Kippen des Reaktor-Behälters (21) in jeweils beide Richtungen umfasst, also über die Neigungswinkel $(+\alpha_{max})$ - 0° - $(-\alpha_{max})$ - 0° - $(+\alpha_{max})$;
e) eine Einrichtung zur Beschickung des Reaktor-Behälters (21) mit einer Dispergierflüssigkeit, die Schwefelsäure mit gelöstem Eisen enthält mit S-$Fe^{3+}$ = 5 bis 30 g/l und einem pH-Wert von 1 bis 3, und welche bioorganische Komplexe aus $Fe^{3+}$ und Exo-Polymeren enthält, mit einem zugeordneten Bakterien-Verband von Acidithiobacillus ferrooxidans, Acidithiobacillus thiooxidans, Leptospirillum ferrooxidans und Ferroplasma acidiphilum;
f) eine Einrichtung zur Regelung der Temperatur T der Lösung der bioorganischen Eisenkomplexe in dem Dispergiermittel in einem Bereich von 10 °C bis 60 °C.

13. Vorrichtung nach Anspruch 12, **gekennzeichnet durch** ein Mahlwerk, insbesondere Kugelmühlen, oder eine Ultraschall-Einrichtung, insbesondere ein Ultraschall-Bad ,um das Nanopulver nach der Reinigung zu desagglomerieren.

14. Vorrichtung nach Anspruch 12 oder 13, **gekennzeichnet durch** einen Vakuumofen, um das Nanopulver bei einer Temperatur von 90 °C bis 130 °C, vorzugsweise bei einer Temperatur von 100 °C bis 120 °C, sowie unter einem Vakuum von 1 mbar bis 50 mbar, vorzugsweise von 2 mbar bis 20 mbar, insbesondere von 5 mbar bis 15 mbar, zu trocknen, und/oder durch einen belüfteten Muffelofen, in den das Wolfram-Nanopulver, insbesondere nach der Desagglomeration, bei einer Temperatur von 550 °C bis 700 °C zum Trocknen ladbar ist, so dass sich das Wolfram-Nanopulver zu $WO_3$ oxidiert, vorzugsweise mit einem monoklinen Gitter und einer Größe der Nanopartikel von 150 nm bis 500 nm.

15. Vorrichtung nach einem der Ansprüche 12 bis 14, **dadurch gekennzeichnet, dass** der Bakterien-Verband von Acidithiobacillus ferrooxidans, Acidithiobacillus thiooxidans, Leptospirillum ferrooxidans und Ferroplasma acidiphilum auf einem festen porösen Träger, insbesondere auf Aktivkohle oder auf Aktivkohle mit Glaukonit, immobilisiert ist.

## Claims

1. Method for the production of a powder consisting of particles of tungsten or of tungsten compounds with a particle size in the nano, micron or submicron range, especially of W, $WO_3$, WC or WC-TiC, WC-TaC or WC-TiC-TaC, through recycling of products of heavy metals' or hard metals' alloys in at least one reactor vessel (21) containing a dispersion, **characterized in that**

a) the degradation of the products is carried out under rotation of the reactor vessel (21), wherein the rotation speed can be controlled and adjusted and is in a range from 0 and 90 rpm according to a preset algorithm, wherein within the preset algorithm the maximum inclination angle ($\alpha_{max}$) of the reactor vessel (21) is between 0° and 40° and the rate of change of the inclination angle (a) is in a range from 0,5 to 2 complete cycles per minute, whereby a complete cycle of the program comprises one inclination of the reactor vessel (21) in each of the both directions, running through the inclination angles $(+\alpha_{max})$ - 0° - $(-\alpha_{max})$ - 0° - $(+\alpha_{max})$;

b) the sulfuric acid with dissolved iron is used as the dispersing liquid, with S-$Fe^{3+}$ = 5 up to 30 g/L and pH value from 1 to 3, comprising further bioorganic complexes of $Fe^{3+}$ and exo-polymers;

c) with an associated bacteria community of acidithiobacillus ferrooxidans, acidithiobacillus thiooxidans, leptospirillum ferrooxidans and ferroplasma acidiphilum;

d) wherein the ratio of the volume $V_{n,o}$ of the products with heavy or/and hard metals' alloys to the volume $V_{b,r}$ of the dispersing liquid is 1 : $4 \leq V_{b,r} / V_{n,o} \leq 1 : 0,5$;

e) wherein the temperature T of the solution of the bioorganic iron complexes in the dispersing liquid is kept in a range from 10 °C to 60 °C;

f) wherein, subsequently, ions of Ni-Fe, Ni-Fe-Cu and/or Co-Fe are released and isolated from the resulting suspension of nano, micron and/or submicron particles in the bioorganic solution, wherein the isolation of Ni, Cu and/or Co leads to a change of the pH value;

g) wherein, after cleaning, the nano powder undergoes a desagglomeration process by ball mills or with ultrasound, with an stabilization solution if necessary;

h) and is subsequently dried in a vacuum oven at a temperature from 90 °C to 130 °C and under vacuum from 1 mbar to 50 mbar.

2. Method according to claim 1, **characterized in that** the tungsten nano powder is dried after the desagglomeration by means of ultra sound in a solution without stabilizers in a vented muffle furnace, loaded at a temperature from 550 °C to 700 °C, wherein said nano powder is oxidized to $WO_3$, having a monoclinic lattice and a size of the nano particles from 150 nm to 500 nm.

3. Method according to claim 1 or 2, **characterized in that** the bacteria community of acidithiobacillus ferrooxidans, acidithiobacillus thiooxidans, leptospirillum ferrooxidans and ferroplasma acidiphilum is immobilized on a porous solid support, especially on activated carbon or on activated carbon with glauconite.

4. Method according to one of claims 1 to 3, **characterized in that** the exo-polymer is produced by the bacteria extracellular on the outer side on the cell wall.

5. Method according to one of the preceding claims, **characterized in that** in step a), the rotation of the reactor vessel (21) is carried out with a speed in the range between 0 and 60 rpm.

6. Method according to one of the preceding claims, **characterized in that** the dispersing fluid comprises S-$Fe^{3+}$ in a concentration from 7 to 20 g/L.

7. Method according to one of the preceding claims, **characterized in that** the dispersing fluid has a pH value from 1,5 to 2,5.

8. Method according to one of the preceding claims, **characterized in that** the ratio of the volume $V_{n,o}$ of the products with heavy and/or hard metals' alloys to the volume $V_{b,r}$ of the dispersing fluid is at 1 : $3 \leq V_{b,r} / V_{n,o} \leq 1 : 1$, especially in a range of 1 : $2,5 \leq V_{b,r} / V_{n,o} \leq 1 : 1,5$, particularly at $V_{b,r} / V_{n,o} \leq 1 : 2$.

9. Method according to one of the preceding claims, **characterized in that** the temperature T of the solution of bioorganic iron complexes in the dispersing fluid is kept in a range from 18 °C to 50 °C.

10. Method according to one of the preceding claims, **characterized in that** in step h), the temperature of the vacuum oven is kept in a range from 100 °C to 120 °C.

11. Method according to one of the preceding claims, **characterized in that** in the step h), the vacuum in the vacuum oven is kept in a range from 8 mbar to 20 mbar, especially in a range from 5 mbar to 10 mbar.

12. Apparatus for the production of a powder consisting of particles of tungsten or of tungsten compounds with a particle size in the nano, micron or submicron range, especially of W, $WO_3$, WC, WC-TiC, WC-TaC or WC-TiC-TaC, through recycling of products of heavy metals' or hard metals' alloys, with at least one reactor vessel (21) containing a dispersion, which is for example rotationally symmetric, preferably oblong or elongated, for degradation of the products, especially in accordance with the method according to one of the preceding claims, **characterized by**

    a) a device for rotating the reactor vessel (21) about an axis of rotation, preferably about its longitudinal axis, the allowable number of revolutions of the reactor vessel (21) being in a range between 0 and 90 rpm;

b) a device for varying the maximum inclination angle ($\alpha_{max}$) of the rotation axis of the reactor vessel (21), especially its longitudinal axis, between 0° and 40°;

c) a device for controlling the number of rotations of the reactor vessel (21), at least in a range between 0 and 90 rpm;

d) a device for controlling and cyclic variation of the inclination angle (a) of the axis of rotation or the longitudinal axis of the reactor vessel (21) in such a way that the rate of change of the inclination angle (a) is in a range between 0,5 and 2 complete cycles per minute, wherein a complete cycle of the program comprises a single tilting of the reactor vessel (21) in each of the both directions, running through the inclination angles ($+\alpha_{max}$) - 0° - ($-\alpha_{max}$) - 0° - ($+\alpha_{max}$);

e) a device for loading the reactor vessel (21) with a dispersing fluid comprising sulfuric acid and dissolved iron with S-$Fe^{3+}$ = 5 to 30 g/L and the pH value from 1 to 3, and comprising bioorganic complexes from $Fe^{3+}$ and exo-polymers, with aassociated bacteria community of acidithiobacillus ferrooxidans, acidithiobacillus thiooxidans, leptospirillum ferrooxidans and ferroplasma acidiphilum;

f) a device for regulating the temperature T of the solution of bioorganic iron complexes in the dispersing fluid in a range from 10 °C to 60 °C.

13. Apparatus according to claim 12, **characterized by** a grinding mechanism, especially ball mills, or an ultrasonic device, especially an ultrasonic bath, for desagglomeration of the nano powder after cleaning.

14. Apparatus according to claim 12 or 13, **characterized by** a vacuum oven for drying the nano powder at a temperature from 90 °C to 130 °C, preferably at a temperature from 100 °C to 120 °C, as well as under a vacuum from 1 mbar to 50 mbar, preferably from 2 mbar to 20 mbar, especially from 5 mbar to 15 mbar, and/or by a vented muffle furnace, into which the tungsten nano powder can be loaded for drying, especially after the desagglomeration, at a temperature from 550 °C to 700 °C, so that the tungsten nano powder is oxidized to $WO_3$, preferably having a monoclinic lattice and a size of the nano particles from 150 nm to 500 nm.

15. Apparatus according to one of claims 12 to 14, **characterized in that** the bacteria community of acidithiobacillus ferrooxidans, acidithiobacillus thiooxidans, leptospirillum ferrooxidans and ferroplasma acidiphilum is immobilized on a porous solid support, especially on activated carbon or on activated carbon with glauconite.

**Revendications**

1. Procédé de production d'une poudre à partir de particules de tungstène ou de liaisons de tungstène ayant une taille de particule située dans la plage de nano, micron ou submicron, en particulier de W, $WO_3$, WC ou WC-TIC, WC-TaC ou WC-TiC-TaC, par recyclage de produits en alliages de métaux lourds ou de métaux durs dans au moins une cuve de réacteur (21) contenant un liquide de dispersion, **caractérisé en ce que**

a) la dégradation des produits est effectuée à une rotation de la cuve de réacteur (21) à une vitesse de rotation réglable contrôlée selon un algorithme prédéfini dans une plage comprise entre 0 et 90 tr/min, **en ce que** dans le cadre de l'algorithme prédéfini l'angle d'inclinaison maximal ($\alpha_{max}$) de la cuve de réacteur (21) est situé entre 0° et 40° et le taux de correction de l'angle d'inclinaison ($\alpha$) est situé dans une plage comprise entre 0,5 et 2 cycles complets par minute, **en ce qu'**un cycle complet du programme comprend le basculement unique de la cuve de réacteur (21) respectivement dans deux directions, c'est-à-dire au-delà de l'angle d'inclinaison ($+\alpha_{max}$) - 0° - ($-\alpha_{max}$) - 0° - ($+\alpha_{max}$) ;

b) **en ce que** de l'acide sulfurique avec du fer dissous est utilisé comme liquide dispersant avec S-$Fe^{3+}$ = 5 à 30 g/l et une valeur pH de 1 à 3, et qui contient des complexes bio-organiques de Fe3+ et d'exopolymères ;

c) avec une association bactérienne correspondante d'acidithiobacillus ferrooxidans, acidithiobacillus thiooxidans, leptospirillum ferrooxidans et ferroplasma acidiphilum ;

d) **en ce que** la proportion entre le volume $V_{n.o}$ des produits avec des alliages de métaux lourds et/ou de métaux durs par rapport au volume $V_{b.r}$ du liquide de dispersion est de 1 : 4 $\leq V_{b.r} / V_{n.o} \leq$ 1 : 0,5 ;

e) **en ce que** la température T de la solution des complexes ferreux bio-organiques dans l'agent dispersant est maintenue dans une plage comprise entre 10 °C et 60 °C ;

f) **en ce qu'**ensuite des ions de Ni-Fe, Ni-Fe-Cu et/ou Co-Fe sont cédés et séparés de la suspension constituée de particules nano, micron et/ou submicron dans la solution bio-organique, **en ce que** la séparation de Ni, Cu et/ou Co modifie la valeur pH ;

g) **en ce que** la nanopoudre après nettoyage est soumise à une opération de désagglomération avec des broyeurs à billes ou aux ultrasons, le cas échéant, avec une solution stabilisante ; h) et est séchée ensuite dans un four à vide à une température de 90 °C à 130 °C ainsi que sous un vide de 1 mbar à 50 mbar.

**2.** Procédé selon la revendication 1, **caractérisé en ce que** la nanopoudre de tungstène est chargée après désagglomération au moyen d'ultrasons dans une solution sans stabilisateur pour le séchage dans un four à moufle ventilé à une température de 550 °C à 700 °C, **en ce que** la nanopoudre s'oxyde sous la forme de $WO_3$, avec un réseau monoclinique et une taille de nanoparticule de 150 nm à 500 nm.

**3.** Procédé selon la revendication 1 ou 2, **caractérisé en ce que** l'association bactérienne d'acidithiobacillus ferrooxidans, acidithiobacillus thiooxidans, leptospirillum ferrooxidans et ferroplasma acidiphilum est immobilisée sur un support poreux solide, en particulier sur du charbon actif ou sur du charbon actif avec de la glauconite.

**4.** Procédé selon l'une des revendications 1 à 3, **caractérisé en ce que** l'exopolymère est produit par les bactéries extracellulairement vers l'extérieur sur la paroi cellulaire.

**5.** Procédé selon l'une des revendications précédentes, **caractérisé en ce que** la rotation de la cuve de réacteur (21) est effectuée à l'étape a) avec un nombre de tours dans une plage comprise entre 0 et 60 tr/min.

**6.** Procédé selon l'une des revendications précédentes, **caractérisé en ce que** le liquide dispersant présente une concentration S-$Fe^{3+}$ = 7 à 20 g/l.

**7.** Procédé selon l'une des revendications précédentes, **caractérisé en ce que** le liquide dispersant présente une valeur pH de 1,5 à 2,5.

**8.** Procédé selon l'une des revendications précédentes, **caractérisé en ce que** la proportion entre le volume $V_{n.o}$ des produits avec des alliages de métaux lourds et/ou de métaux durs par rapport au volume $V_{b.r}$ du liquide de dispersion est 1 : 3 ≤ $V_{b.r}$ / $V_{n.o}$ ≤ 1 : 1, de préférence dans une plage de 1 : 2,5 ≤ $V_{b.r}$ / $V_{n.o}$ ≤ 1 : 1,5, en particulier de $V_{b.r}$ / $V_{n.o}$ ≤ 1 : 2.

**9.** Procédé selon l'une des revendications précédentes, **caractérisé en ce que** la température T de la solution des complexes ferreux bio-organiques dans l'agent dispersant est maintenue dans une plage comprise entre 18 °C et 50 °C.

**10.** Procédé selon l'une des revendications précédentes, **caractérisé en ce que** la température du four à vide est maintenue à l'étape h) dans une plage comprise entre 100 °C et 120 °C.

**11.** Procédé selon l'une des revendications précédentes, **caractérisé en ce que** le vide dans le four à vide est maintenu à l'étape h) dans une plage comprise entre 8 mbar et 20mbar, en particulier dans une plage comprise entre 5 mbar et 10 mbar.

**12.** Dispositif de production d'une poudre à partir de particules de tungstène ou de liaisons de tungstène ayant une taille de particule située dans la plage de nano, micron ou submicron, en particulier de W, $WO_3$, WC ou WC-TIC, WC-TaC ou WC-TiC-TaC, par recyclage de produits en alliages de métaux lourds ou de métaux durs, avec au moins une cuve de réacteur (21) par exemple symétrique en rotation, de préférence oblongue ou allongée, contenant un liquide de dispersion pour la dégradation des produits, en particulier suivant le procédé selon l'une des revendications précédentes, **caractérisé par**

a) un équipement pour déplacer la cuve de réacteur (21) en rotation autour d'un axe de rotation, de préférence autour de son axe longitudinal, à un nombre de tours admissible de la cuve de réacteur (21) au moins dans une plage comprise entre 0 et 90 tr/min ;

b) un équipement pour varier entre 0° et 40° l'angle d'inclinaison maximal ($\alpha_{max}$) de l'axe de rotation de la cuve de réacteur (21), en particulier son axe longitudinal ;

c) un équipement pour la commande du nombre de tours de la cuve de réacteur (21) au moins dans une plage comprise entre 0 et 90 tr/min ;

d) un équipement pour la commande et la modification cyclique de l'angle d'inclinaison ($\alpha$) de l'axe de rotation ou de l'axe longitudinal de la cuve de réacteur (21) de telle façon que le taux de correction de l'angle d'inclinaison ($\alpha$) est dans une plage comprise entre 0,5 et 2 cycles complets par minute, en ce qu'un cycle complet du programme comprend le basculement unique de la cuve de réacteur (21) respectivement dans deux directions, c'est-à-dire au-delà de l'angle d'inclinaison ($+\alpha_{max}$) - 0° - ($-\alpha_{max}$) - 0° - ($+\alpha_{max}$) ;

e) un équipement pour le chargement de la cuve de réacteur (21) avec un liquide dispersant, qui comprend de l'acide sulfurique avec du fer dissous avec S-$Fe^{3+}$ = 5 à 30 g/l et une valeur pH de 1 à 3, et qui contient des complexes bio-organiques de Fe3+ et d'exopolymères, avec une association bactérienne correspondante d'acidithiobacillus ferrooxidans, acidithiobacillus thiooxidans, leptospirillum ferrooxidans et ferroplasma acidiphilum ;

f) un équipement pour la régulation de la température T de la solution des complexes ferreux bio-organiques dans l'agent dispersant dans une plage comprise entre 10 °C et 60 °C.

13. Dispositif selon la revendication 12, **caractérisé par** un broyeur, en particulier des broyeurs à billes, ou un équipement à ultrasons, en particulier un bain à ultrasons, pour désagglomérer la nanopoudre après nettoyage.

14. Dispositif selon la revendication 12 ou 13, **caractérisé par** un four à vide pour sécher la nanopoudre à une température comprise entre 90 °C et 130 °C, de préférence à une température comprise entre 100 °C et 120 °C, ainsi que sous un vide de 1 mbar à 50 mbar, de préférence de 2 mbar à 20 mbar, en particulier de 5 mbar à 15 mbar, et/ou par un four à moufle ventilé dans lequel la nanopoudre de tungstène peut être chargée pour le séchage, en particulier après l'opération de désagglomération, à une température comprise entre 550 °C et 700 °C de façon à ce que la nanopoudre de tungstène s'oxyde sous forme de $WO_3$, de préférence avec un réseau monoclinique et une taille de nanoparticule de 150 nm à 500 nm.

15. Dispositif selon l'une des revendications 12 à 14, **caractérisé en ce que** l'association bactérienne d'acidithiobacillus ferrooxidans, acidithiobacillus thiooxidans, leptospirillum ferrooxidans et ferroplasma acidiphilum est immobilisée sur un support poreux solide, en particulier sur du charbon actif ou sur du charbon actif avec de la glauconite.

# Fig.1

a) Vorbereitung bioorganischer
Lösungen mit Eisenkomplex

b) Gewinnung eines Pulvers aus Mikro-, Submikro- und/oder
Nanopartikel („Nanopulver") sowie Gewinnung von Ni-Fe und/oder
Ni-Fe-Cu- Lösungen in rotierenden, geneigten Behältern unter
Recycling von Produkten aus Metallen oder Legierungen wie
W, Ni- (Fe, Cu), WC-Co, WC-TiC, WC-TiC-TaC

c) Zentrifugieren und Abtrennen des Nanopulvers sowie der Ni-Fe-,
Ni-Fe-Cu- und/oder Co-Fe-Lösungen

d) Spülen des Nanopulvers mit einer
schwachen Lösung von Säure und
destilliertem Wasser

d.1) Abscheiden von Nickel, Kupfer, Eisen
und/oder Kobalt sowie Regeneration
der Lösung für die Vorbereitung der
bioorganischen Lösung

e) Aufteilen des Nanopulvers nach
Partikelgröße ( <1 Mikron )

d.2) Trocknen des Nickel-, Kupfer-
und/oder Eisenpulvers

h) Lufttrocknung des Wolfram-Nanopulvers in einem Muffelofen bei
540-740°C, um Wolframoxid-
Nanopulver zu erhalten

f) Zerstörung von Agglomeraten des
Nanopulvers;
Beschichtung mit Tensiden

g) Trocknen der W-, WC-, WC-TiC und/
oder WC-TiC-TaC- Pulver in einem
Vakuumofen unter einer Füllung mit
Argon

i) Abfüllen des Wolframoxid-Nano-
Pulvers in Behälter

Fig.2

EP 3 138 932 B1

16

Fig.3a

Fig.3b

Fig. 4a

**Karteninformation:**

| | |
|---|---|
| Namen: | Wolfram |
| Formel: | W |
| PDF-Nr.: | 4-806 |
| Qualität: | Stern |
| Unterdateien: | Anorganische Legierung NBS CP FOR EDU |

**Zellen- und Symmetrie-Information:**

| | | | | |
|---|---|---|---|---|
| System: | Kubisch | **Raumgruppe:** | Im3m | (Nr. 229) |
| a: | 3,1648 | | | |
| Dichte (Dx): | 19,265 | Z: | 2 | |

**Instrumenten-Information:**

| | | | | | |
|---|---|---|---|---|---|
| Strahlung: | CuKa1 | **Wellenlänge:** | 1,5404 | **Filter:** | Ni |
| Instrument (d) | unbekannt | | | | |
| Instrument (I): | Diffraktometer | I-Typ | unbekannt | | |
| I/Icor: | 18,00 | | | | |

**Kommentare und zusätzliche Informationen**

| | |
|---|---|
| Farbe: | Metallisch grau |
| Quelle: | Das Muster wurde bei der Westinghouse Electric Corp. aufbereitet |
| Vorbereitung | Die Analyse des Musters zeigt O2 0,04 %, Ni 0,05 %, Mo, Fe und jeweils 0,01 % |
| Temperatur: | Das Muster wurde bei 26° C genommen |
| Allgemein: | Merck Index, 8. Ausgabe, Seite 1087 |

**Literaturangaben:**

| | |
|---|---|
| Allgemein: | Swanson, Tatge Natl. Bur. Stand (US) Circ. 539 I 28 (1953) |

Fig. 4b

<u>Karteninformation:</u>

| | |
|---|---|
| **Namen:** | Wolframoxid |
| **Formel:** | WO₃ |
| **PDF-Nr.:** | 20-1324 |
| **Qualität:** | indiziert |
| **Unterdateien:** | Anorganische Legierung FOR COR |

<u>Zellen- und Symmetrie-Information:</u>

| **System:** | monoklin | **Raumgruppe:** | P2/m | (Nr. 10) | |
|---|---|---|---|---|---|
| **a:-** | -7,28 | **b:-** | -7,5 | **c:-** | -3,83 |
| **ß:** | 91 | | | | |
| **Z:** | 1 | | | | |

<u>Instrumenten-Information:</u>

| **Strahlung:** | CuKa | **Wellenlänge:** | 1,5418 | **Filter:** | Ni |
|---|---|---|---|---|---|
| **Instrument (d)** | unbekannt | | | | |
| **Instrument (I):** | Diffraktometer | **I-Typ** | unbekannt | | |

<u>Kommentare und zusätzliche Informationen</u>

| **Farbe:** | gelb |
|---|---|
| **Allgemein:** | stabilisiert mit 2 % Nb2 O5 |

<u>Literaturangaben:</u>

| **Allgemein:** | Roth, Waring J. Res. Natl. Bur. Stand., Sect. A 70 281 (1966 |
|---|---|

# Fig. 5

Fig. 6a

**Karteninformation:**

| | |
|---|---|
| **Namen:** | Wolframcarbid |
| | Unbenanntes Mineral, syn [NR] |
| **Formel:** | W C |
| **PDF-Nr.:** | 51-939 |
| **Qualität:** | Stern |

**Zellen- und Symmetrie-Information:**

| | | | | |
|---|---|---|---|---|
| **System:** | hexagonal | **Raumgruppe:** | P.-6m2 | (Nr. 187) |
| **a:-** | 2.90631 | **c:-** | 2.83754 | |
| **Dichte (Dx):** | 15,660 | **Z:** | 1 | |

**Instrumenten-Information:**

| | | | | | |
|---|---|---|---|---|---|
| **Strahlung:** | CuKa1 | **Wellenlänge:** | 1,5406 | **Filter:** | Ni |
| **Instrument (d)** | Diffraktometer | **Ausgeschaltet:** | 8,8 | **Standard:** | Si |
| **Instrument (l):** | Diffraktometer | **I-Typ** | Spitzenhöhen | | |
| **I/Icor:** | 14,94 | | | | |

**Kommentare und zusätzliche Informationen**

| | |
|---|---|
| **Farbe:** | schwarz |
| **Schmelzpunkt:** | 2825 |
| **Quelle:** | Handelsübliche Quelle - Cerac (T-1173) |
| **Analyse:** | Chemische Analyse (Gew.-%): C 6.153, V 0,08, Ca <0,01 |
| **Muster:** | siehe ICSD 15406 (PDF 72-97); ICSD 22258 (PDF 73-471). |
| **Muster:** | als Ersatz für 25-1047 |
| **Quelle:** | Als Mineral von Mengyin, Shadong und Danba, Sichuan China beschrieben Zianhong, Z. Guojie, Zhaohni, Acta Mineral. Sinica, 6 344-349 (1986) |

**Literaturangaben:**

| | |
|---|---|
| **Allgemein:** | Mayo, W., H&M Analytical Services, Inc., Allentown, NJ. USA Grant-Aid |
| **Elementarzelle:** | Leciejewicz, J. Acta Crystallogr. 14 200 (1961) |

Fig. 6b

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011000955 A1
- JP 2009249415 A

**In der Beschreibung aufgeführte Nicht-Patentliteratur**

- **KOJIMA T. et al.** Recycling process of WC-Co cermets by hydrothermal treatment. *Journal of Materials Science,* vol. 40 (19), 5167-5172